# EUROPEAN PATENT APPLICATION

(11) **EP 3 951 395 A1**
(43) Date of publication of application: **09.02.2022**
(21) Application number: 20776974.6
(22) Date of filing: 23.01.2020
(51) Int. Cl.: G01N 35/00

(54) **AUTOMATIC ANALYSIS DEVICE**

(30) Priority: 27.03.2019 JP 2019060018
(71) Applicant: Hitachi High-Tech Corporation, Minato-ku Tokyo 105-6409 (JP)
(72) Inventor: SHIMIZU, Kanoh, Tokyo 105-6409 (JP); YAGI, Kenichi, Tokyo 105-6409 (JP); ONOSE, Toma, Tokyo 105-6409 (JP)
(74) Representative: Strehl Schübel-Hopf & Partner
(86) International application number: PCT/JP2020/002299
(87) International publication number: WO 2020/195071

(57) **Abstract**

Provided is an automatic analyzer capable of facilitating maintenance work and adjustment work. Automatic analyzers 100 and 110 are operable by main body software that controls operation of the automatic analyzers 100 and 110 and attached software that is independent of the main body software and is used during maintenance of machines that configure the automatic analyzers 100 and 110. The attached software and the main body software respectively hold specific identification information that specifies an own version. At least one of the attached software and the main body software holds corresponding identification information of a counterpart side software corresponding to current time. Operation units 103 and 113 specify the attached software suitable for the main body software from a plurality of pieces of attached software by comparing the specific identification information and the corresponding identification information.

## Description

### Technical Field

The present invention relates to an automatic analyzer that performs quantitative and qualitative analysis of biological samples such as blood and urine.

### Background Art

In a case where both biochemical analysis in biochemical analysis modules and immunoassay in immunoassay modules are performed for the same specimen, for the purpose of preventing an effect of the specimen carry-over from the biochemical analysis modules to the immunoassay, PTL 1 discloses that the specimen is conveyed to any one of the immunoassay modules by a conveying line and immunoanalyzed, if dispensing of the specimen is completed, the specimen is conveyed to a specimen rack standby unit by the conveying line and temporarily waits until the analysis result is obtained, as a result of the analysis, in a case where re-examination is necessary, the specimen returns to the original place by a return conveying line without being conveyed to any subsequent biochemical analysis modules and immunoanalyzed again, and on the contrary, in a case where re-examination is not necessary, the rack is conveyed to the subsequent biochemical analysis modules without returning to the original place for the biochemical analysis.

### Citation List

### Patent Literature

PTL 1: JP-A-2000-074925

### Summary of Invention

### Technical Problem

Automatic analyzers that automatically perform quantitative and qualitative analysis of a specimen are widely used in large hospitals and clinical examination centers where many patient specimens need to be processed for a short period of time. For the automatic analyzers, devices having various processing abilities such as large, medium, and small-sized devices depending on the processing ability have been developed.

As one example of the automatic analyzers, PTL 1 discloses a technology. In such automatic analyzers, a regular maintenance operation is necessary to ensure higher reliability. On the other hand, a high processing ability and a speedy result output are demanded.

For this reason, especially, in the automatic analyzer used in a facility where a large number of patient specimens are processed in one day, it is desired not to stop the device as much as possible in order to output a measurement result as quickly as possible. As a part of the technology for the above, it is desired to complete the maintenance operation in a short time.

The same applies to a case of adding a new function to an analyzing unit or a conveying unit that conveys a sample of the automatic analyzer, or a case of changing the settings.

In order to simplify the maintenance or various adjustments of the automatic analyzer, there is provided attached software intended for the maintenance or adjustments of individual unit or machines that configures the analysis system.

The attached software can be operated at the terminal different from the machine that configures the automatic analyzer, especially, the operation unit that controls the overall operation of the automatic analyzer. The attached software communicates directly with individual device via a network and enables maintenance.

Here, there may be a case where a user who uses the attached software most is a service person in charge of the maintenance of the automatic analyzers in multiple customers' places.

In this case, the service person visits the customer's place, and then first selects the version of the attached software that is suitable for the automatic analyzer. Thereafter, the PC operated by the service person and the automatic analyzer are connected to each other via a network, and then settings of the network or settings of the device configuration need to be performed. The maintenance can be started only after the settings are completed. For this reason, a lot of time and effort are required, and a technology has been demanded by which the burden on the service person can be reduced and the maintenance can be completed more quickly.

An object of the invention is to provide an automatic analyzer that can facilitate maintenance work or adjustment work.

### Solution to Problem

The invention includes a plurality of means for solving the above problem. As one example thereof, an automatic analyzer that includes an analyzing unit that executes specimen analysis; and a control unit that controls operation of machines in the device including the analyzing unit, in which the automatic analyzer is operable by main body software that controls operation of the automatic analyzer and attached software that is independent of the main body software and is used during maintenance of the machines that configures the automatic analyzer, the attached software and the main body software respectively hold specific identification information that specifies an own version, at least one of the attached software and the main body software holds corresponding identification information of a counterpart side software corresponding to current time, and the control unit specifies the attached software suitable for the main body software from a plurality of pieces of attached software by comparing the specific identification information and the corresponding identification information.

### Advantageous Effects of Invention

According to the invention, the maintenance work and adjustment work can be further facilitated. Problems, configurations, and effects other than the above will be clarified by the description of the following embodiments.

### Brief Description of Drawings

[FIG. 1]FIG. 1 is a schematic diagram showing an example of a configuration of an automatic analyzer according to an embodiment of the invention.
[FIG. 2]FIG. 2 is a schematic diagram showing an example of a configuration of an analyzing unit in the automatic analyzer according to the embodiment of the invention.
[FIG. 3] FIG. 3 is a diagram showing an example of software provision time and an identifier in the automatic analyzer according to the embodiment.
[FIG. 4]FIG. 4 is a diagram showing a correspondence relationship between main body software and attached software shown in FIG. 3.
[FIG. 5] FIG. 5 is a flowchart showing a flow of specifying the attached software from information on the main body software executed in the automatic analyzer according to the embodiment.
[FIG. 6] FIG. 6 is a diagram showing an example of a Venn diagram that specifies the attached software from the information on the main body software in the automatic analyzer according to the embodiment.
[FIG. 7]FIG. 7 is a diagram showing another example of a Venn diagram that specifies the attached software from information on the main body software in the automatic analyzer according to the embodiment.
[FIG. 8]FIG. 8 is a diagram showing an example of setting information of the attached software in the automatic analyzer according to the embodiment.
[FIG. 9] FIG. 9 is a diagram showing an example of functions of the attached software in the automatic analyzer according to the embodiment.
[FIG. 10] FIG. 10 is a diagram showing an example of update timing of the setting information of the attached software in the automatic analyzer according to the embodiment.
[FIG. 11]FIG. 11 is a diagram showing a workflow for maintenance of an automatic analyzer in the related art.
[FIG. 12]FIG. 12 is a diagram showing a workflow for maintenance of the automatic analyzer according to the embodiment.

### Description of Embodiments

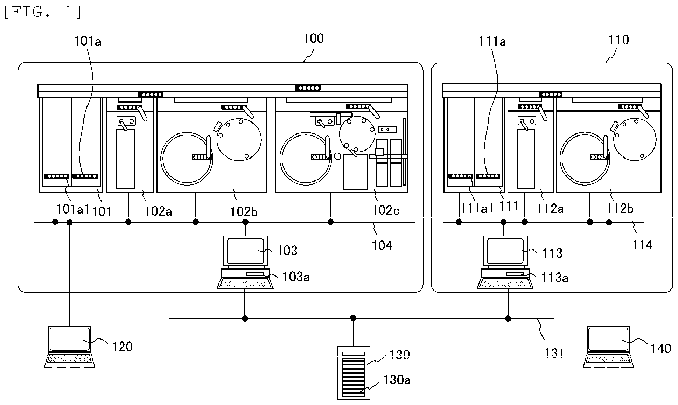

Embodiments of an automatic analyzer of the invention will be described using FIGS. 1 to 12.

First, an overall configuration of an automatic analyzer of a preferred embodiment of the invention will be described using FIG. 1. FIG. 1 is an explanation diagram showing an example of a configuration of an automatic analyzer according to an embodiment of the invention. The explanation diagram shown in FIG. 1 schematically shows a plurality of automatic analyzers 100 and 110, attached software PCs 120 and 140, and an information managing PC 130.

As shown in FIG. 1, the automatic analyzer 100 according to the embodiment includes a sample conveying unit 101 connected to a network 104, analyzing units 102a, 102b, and 102c, and an operation unit 103. The automatic analyzer 110 includes a sample conveying unit 111 connected to a network 114, analyzing units 112a and 112b, and an operation unit 113.

In the automatic analyzer 100, the operation unit 103 is connected to an information managing PC 130 via a network 131, and in the automatic analyzer 110, the operation unit 113 is connected to the information managing PC 130 via the network 131. The number of the automatic analyzers connected to the information managing PC 130 is not limited and may be one or more.

Labels or barcodes, on which specimen ID indicating attribute information (reception number, patient name, requested analysis items, and the like) related to an examination sample (specimen) such as blood is recorded, are attached to specimen containers 101a1 and 111a1 held by specimen racks 101a and 111a. Tags indicating rack ID showing rack identification information such as a rack number are attached to the specimen racks 101a and 111a.

The specimen racks 101a and 111a placed on the sample conveying units 101 and 111 are respectively conveyed into devices by sample conveying units 101 and 111. At that time, information related to the specimen ID or the specimen rack ID are read by an ID reading unit (not shown), and the information is transmitted to the operation unit 103 or the operation unit 113.

The sample conveying units 101 and 111 convey the specimen racks 101a and 111a input from a specimen rack input unit (not shown) to the analyzing units 102a, 102b, and 102c or the analyzing units 112a and 112b which become the target according to the analysis request made by a user such as an examination technician or doctor. The specimen racks 101a and 111a of which analysis is completed in the analyzing units 102a, 102b, and 102c or the analyzing units 112a and 112b, or the specimen racks 101a and 111a of which analysis is not requested are conveyed to a specimen rack recovering unit (not shown).

The analyzing units 102a, 102b, and 102c or the analyzing units 112a and 112b are modules that execute analysis of the specimens in the specimen containers 101a1 and 111a1 held by the specimen racks 101a and 111a conveyed via the sample conveying units 101 and 111. The analyzing units are arranged along the sample conveying units 101 and 111, and are connected to detach from the sample conveying units 101 and 111.

The number of the analyzing units 102a, 102b, and 102c or the analyzing units 112a and 112b can be arbitrarily set, and at least one type of the analyzing unit may be arranged one or more. In the embodiment, in the automatic analyzer 100, the number of modules is 3, and in the automatic analyzer 110, the number of modules is 2. The type of the analyzing unit can be other than the above-described 3.

The sample conveying units 101 and 111 may be arranged one or more, and two or more units can be arranged.

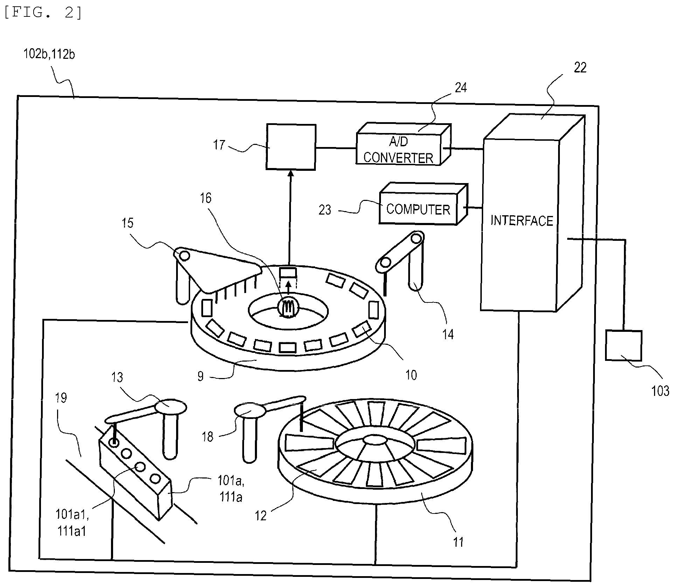

Hereinafter, as one example of the configuration of the analyzing units 102a, 102b, and 102c or the analyzing units 112a and 112b, the analyzing units 102b and 112b for biochemistry that executes analysis of the biochemical analysis items will be described as an example using FIG. 2. FIG. 2 is a schematic diagram of the theoretical overall configuration of the analyzing units 102b and 112b for biochemistry.

In FIG. 2, the analyzing unit 102b includes a reaction disk 9, a reagent disk 11, a rack conveying belt 19, a reagent dispensing probe 18, a specimen dispensing probe 13, a stirring device 14, a light source 16, a multi-wavelength photometer 17, a cleaning device 15, and a computer 23.

The reaction containers 10 are arranged on the circumference of the reaction disk 9. The rack conveying belt 19 that moves the specimen racks 101a and 111a where the specimen containers 101a1 and 111a1 are placed is provided near the reaction disk 9.

The specimen dispensing probe 13 that can rotate and move up and down is provided between the reaction disk 9 and the rack conveying belt 19. The specimen dispensing probe 13 moves around the rotation axis while drawing an arc to dispense a sample from the specimen containers 101a1 and 111a1 to the reaction container 10.

A plurality of reagent bottles 12 can be placed on the circumference of the reagent disk 11. The reagent disk 11 is kept cool and covered with a cover provided with a suction port.

A reagent dispensing probe 18 that can rotate and move up and down is provided between the reaction disk 9 and the reagent disk 11. The reagent dispensing probe 18 moves around the rotation axis while drawing an arc, accesses the inside of the reagent disk 11 by the suction port, and dispenses a reagent from the reagent bottle 12 to the reaction container 10.

Further, a cleaning device 15, a light source 16, a multi-wavelength photometer 17, and a stirring device 14 are arranged around the reaction disk 9. A cleaning tank (not shown) is provided respectively within the operation ranges of the reagent dispensing probe 18, the specimen dispensing probe 13, and the stirring device 14.

The computer 23 is configured by a personal computer equipped with a CPU, memory, and the like, and is connected to mechanisms within the above-described analyzing units 102b and 112b via an interface 22. The computer controls all of the operations of the mechanisms and performs arithmetic processing for obtaining the concentration of a predetermined component in a liquid sample such as blood and urine. The computer 23 is connected to the operation unit 103 or the operation unit 113 via the interface 22.

Returning to FIG. 1, the operation unit 103 or the operation unit 113 is a computer including a CPU, memory, interface, and the like, and executes various controls necessary for operating the machines within the respective automatic analyzers 100 and 110 including the analyzing units 102a, 102b, 102c, 112a, and 112b.

A control of the operation of the machines that configures the automatic analyzers 100 and 110 by the operation units 103 and 113 is performed by reading and executing a program (hereinafter, referred to as main body software) stored in recording devices 103a and 113a or external recording media (not shown) by the CPU.

The "main body software" described in the embodiment is a general term for software for operating the mechanisms that configures one automatic analyzer 100 or automatic analyzer 110.

The automatic analyzers 100 and 110 in the embodiment is independent of the main body software and the main body software, and can be operated by attached software used at the time of maintenance, which will be described below.

The operation unit 103 or the operation unit 113 of the embodiment specifies attached software that is suitable for the main body software from a plurality of pieces of attached software by comparing specific identification information for specifying an own version of the main body software and corresponding identification information of a counterpart side software corresponding to current time. The operation units execute the processing of releasing the specified attached software toward the attached software PCs 120 and 140 operated by the service person. The details thereof will be described below.

The operation unit 103 or the operation unit 113 is further connected with operation equipment composed of a keyboard or mouse for inputting data, a display device for displaying data, a printing device for printing data, and the recording devices 103a and 113a for recording analysis instruction information or measurement result.

The display device is display equipment such as a liquid crystal display that displays analysis result or progress situation of the analysis, and information associated with maintenance in the device including the analyzing units 102a, 102b, and 102c or the analyzing units 112a and 112b.

The recording devices 103a and 113a are recording media such as a semiconductor memory, e.g., a flash memory where data associated with the specimen input into the automatic analyzers 100 and 110 or analysis result are recorded, and a magnetic disk, e.g., an HDD.

Further, the attached software reflecting information on the respective automatic analyzers 100 and 110 is recorded in the recording devices 103a and 113a of the embodiment. The details thereof will be described below.

An information managing PC 130 is a server operated by, for example, a manufacturer or an agency of the automatic analyzers 100 and 110. The information managing PC has a recording device 130a where at least one piece of attached software intended for maintenance of the individual device that configures the automatic analyzer 100 or the automatic analyzer 110 is recorded.

The automatic analyzers 100 and 110 of the embodiment are connected with the attached software PCs 120 and 140 at the time of maintenance by the service person, addition of new functions, and adjustments.

The attached software PC 120 is able to communicate with a sample conveying unit 101, the analyzing units 102a, 102b, and 102c, and the operation unit 103 of the automatic analyzer 100 by being connected to the network 104.

The attached software PC 140 is able to communicate with a sample conveying unit 111, the analyzing units 112a and 112b, and the operation unit 113 of the automatic analyzer 110 by being connected to the network 114. The attached software PC 140 may be the same as or different from the attached software PC 120.

The attached software PCs 120 and 140 are PCs independent of the automatic analyzers 100 and 110, and can participate in the network 104 in the automatic analyzer 100 or the network 114 in the automatic analyzer 110 at any timing when the attached software is used.

The above-described attached software is packaged for each automatic analyzer product, and if the product is different, the type of the attached software is different. Also, the attached software corresponding to one type of the automatic analyzer has different versions for fixing bugs or adding functions.

The above is the configuration of the automatic analyzers 100 and 110.

The automatic analyzer is not limited to a case where a plurality of analyzing units or sample conveying units is included as shown in FIG. 1, and may be configured to include one analyzing unit and one operation unit. In this case, the computer 23 and the operation unit can be integrated.

The analysis processing of a specimen by the above-described automatic analyzers 100 and 110 is generally executed according to the following order. In the following, a case of the analyzing units 102b and 112b will be described as a representative.

An operator gives an analysis instruction to the automatic analyzers 100 and 110 by using the operation unit 103 or the operation unit 113. The analysis instruction is recorded in the recording devices 103a and 113a and transmitted to the target analyzing units 102b and 112b via the operation unit 103 or the operation unit 113. The analyzing units 102b and 112b perform an analysis operation as follows according to the received analysis instruction.

In the analyzing units 102b and 112b, first, a specimen within the specimen containers 101a1 and 111a1 placed on the specimen racks 101a and 111a conveyed close to the reaction disk 9 by the rack conveying belt 19 via the sample conveying units 101 and 111 is dispensed in a predetermined amount to the reaction container 10 on the reaction disk 9 by the specimen dispensing probe 13.

If dispensing of one specimen containers 101a1 and 111a1 is completed, the rack conveying belt 19 moves the specimen racks 101a and 111a so that the next specimen containers 101a1 and 111a1 come directly below the specimen dispensing probe 13. If dispensing of all of the specimen containers 101a1 and 111a1 on the specimen racks 101a and 111a is completed, the specimen racks 101a and 111a are conveyed by the rack conveying belt 19.

The reaction container 10 in which the specimen is dispensed rotationally moves on the reaction disk 9 by the rotation operation of the reaction disk 9. Meanwhile, a reagent used for the analysis is dispensed to the specimen in the reaction container 10 from the reagent bottle 12 on the reagent disk 11 by the reagent dispensing probe 18.

Subsequently, a reaction solution of the specimen and the reagent within the reaction container 10 is stirred by the stirring device 14.

Thereafter, light generated from the light source 16 is transmitted to the reaction container 10 containing the reaction solution and luminous intensity of the transmitted light is measured by the multi-wavelength photometer 17.

An absorbance signal measured by the multi-wavelength photometer 17 is transmitted to the computer 23 via an A/D converter 24 and the interface 22.

From the absorbance signal, based on the analysis method set in advance for each test substance, the computer 23 calculates the calibration curve data from the set concentration data in a case of the standard solution specimen, and calculates concentration data from the calibration curve data obtained by measuring the standard solution specimen, in a case of the patient specimen and the control specimen. The data are transmitted to the operation unit 103 or the operation unit 113 via the interface 22 as the measurement result, after information in which the type of the specimen is encrypted is added thereto.

In parallel with the above, the reaction container 10 of which the analysis is completed is cleaned by the cleaning device 15 and used for the next analysis.

The operation unit 103 or the operation unit 113 records the received measurement result in the recording devices 103a and 113a and outputs the measurement result to the display device or printing device.

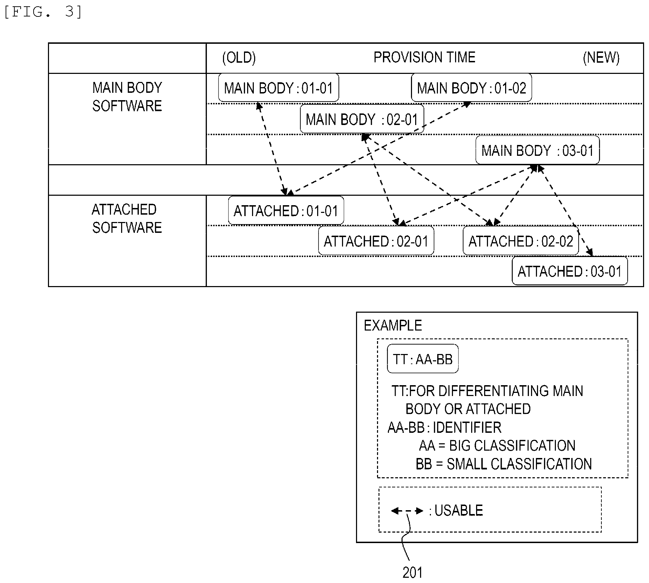

Next, the details of the processing of specifying the attached software or the details of the subsidiary processing in the automatic analyzers 100 and 110 of the embodiment will be described using FIG. 3 and the following drawings.

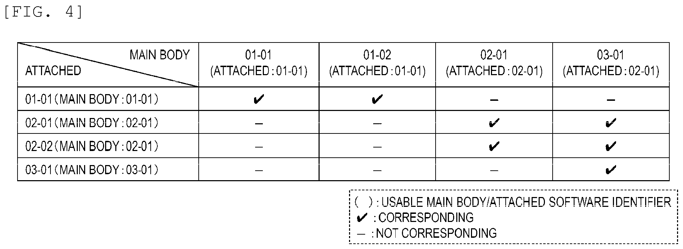

First, the details of the specific identification information or corresponding identification information included in the main body software and the attached software will be described using FIGS. 3 and 4. FIG. 3 is a diagram showing an example of provision time and an identifier of the main body software or the attached software in the automatic analyzer. FIG. 4 is a diagram showing a correspondence relationship between the main body software and the attached software shown in FIG. 3.

The main body software and the attached software that operate the automatic analyzers 100 and 110 of the embodiment respectively holds an identifier configured by specific identification information for uniquely specifying an own version and corresponding identification information for specifying a counterpart side software corresponding to current time. The identifier is updated by addition or modification of functions.

That is, in the embodiment, the main body software holds an identifier in which the minimum identification information (corresponding identification information) of the attached software that can be used by the own machine and identification information (specific identification information) that specifies a version of the main body software that operates the own machine are recorded as the information. In the same manner, the attached software holds the minimum identification information (specific identification information) that specifies a version of the own software and identification information (corresponding identification information) that specifies a version of the main body software that can be operated by the own software are recorded as the information.

The identifier may be the information that is different from or the same as the identification information. In the following, a case where the identifier and the identification information are the same information will be described as an example. In a case where the identifier and the identification information are different information, it is preferable that the identifier and the identification information are uniquely determined.

As shown in FIG. 3, the identifier is defined by a numerical value of which the magnitude can be judged or a character string. The row direction in FIG. 3 corresponds to the functions of the respective software, and the column direction corresponds to the provision times of the respective software.

In FIG. 3, the configuration of the identifier is described by two classifications, which are major two-digit classification and small two-digit classification. However, the configuration of the identifier or the number of digits of the identifier may be one or more, and is not particularly limited.

In the embodiment, at the time of the specification processing, the operation units 103 and 113 determine the software having a large major classification number as the new software, and in a case where the major classification numbers are the same as each other, the operation units determine the software having a large minor classification number as the new software.

FIG. 3 shows the attached software that can be used with respect to the main body software in association with each other by using the lines of an example 201. FIG. 4 is a list of the correspondence relationship between the main body software and the attached software shown in FIG. 3. In FIG. 4, the vertical axis shows the identifier of the attached software, and the horizontal axis shows the identifier of the main body software.

In FIG. 4, the minimum identifier of the main body software or the attached software corresponding to the embodiment is shown within () of FIG. 4.

In the embodiment, it is described that the main body software and the attached software mutually holds the identifier of the counterpart software. However, the identifier of the counterpart software may be held by only the main body software or only the attached software.

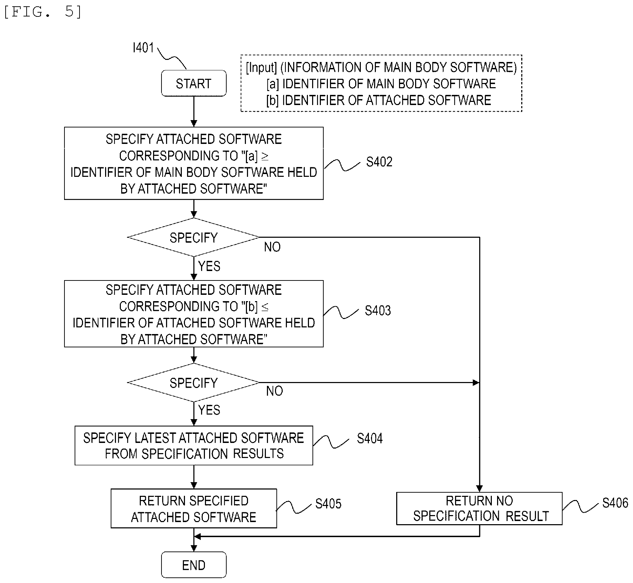

Next, FIGS. 5 to 7 are used to describe a flow of the processing for specifying the latest suitable attached software from a plurality of pieces of attached software by the operation units 103 and 113 operated by the main body software.

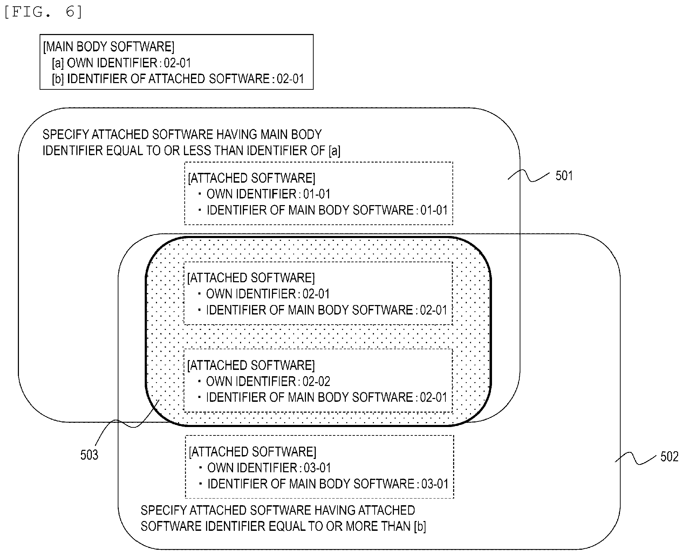

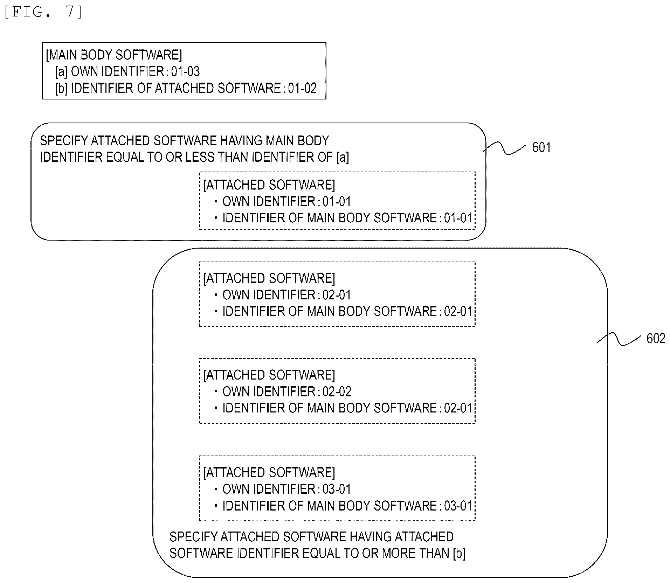

FIG. 5 is a flow chart showing a flow of specifying the attached software from information on the main body software. FIGS. 6 and 7 are diagrams showing an example of a Venn diagram that specifies the attached software from the information on the main body software.

The input 401 in FIG. 5 is an input condition of the flow chart, and designates the identifier of the attached software corresponding to the own identifier of the main body software held by the main body software.

Input 401 of FIG. 5 or each subsequent step thereof will be described in the assumption that the steps are executed in parallel with various warm-up operations executed after the power supply of the automatic analyzers 100 and 110 is turned ON. However, the execution timing is not particularly limited. For example, the steps can be executed when the specific instruction signal is input from the attached software PCs 120 and 140, or after the specific instruction is input by operation of the operation units 103 and 113.

First, the operation units 103 and 113 specify the attached software that matches "the identifier of the main body software ≥ the identifier of the main body software held by the attached software" from the attached software recorded in the recording device 130a of the information managing PC 130, or the recording devices 103a and 113a by comparing the own identifier of the main body software designated by Input 401 with the identifier of the main body software held by the attached software (step S402). For example, the aggregation 501 of FIG. 6 or the aggregation 601 of FIG. 7 are specified by the step S402.

Next, the operation units 103 and 113 specify the attached software that matches "the identifier of the attached software ≤ the identifier of the attached software held by the attached software" from the attached software recorded in the recording device 130a of the information managing PC 130, or the recording devices 103a and 113a by comparing the identifier of the attached software held by the main body software designated by Input 401 with the own identifier of the attached software held by the attached software (step S403). For example, the aggregation 502 of FIG. 6 or the aggregation 602 of FIG. 7 are specified by the step S403.

The embodiment is specified in the order of the step S402 and the step S403, but the order of the steps may be from the step S403 to the step S402 or the steps may be executed in parallel.

Thereafter, in a case where there is a plurality of pieces of attached software specified by the step S402 and the step S403, the operation units 103 and 113 uniquely specify the attached software from the plurality of pieces of attached software (step S404) . In the embodiment, software having the largest identifier in the order of large classification and medium classification is treated as the latest software. If there is no a plurality of pieces of software, the one specified attached software is uniquely specified.

Thereafter, the operation units 103 and 113 return the attached software uniquely specified in the step S404 to the recording devices 103a and 113a in order to release the software toward the attached software PCs 120 and 140 operated by the service person (step S405) . On the other hand, in a case where the attached software is not specified in the step S402 or the step S403, the operation units return to "no specific result" (step S406).

The step S404 may be omitted. In other words, the attached software specified multiple times may return to the recording devices 103a and 113a in order to release the software toward the attached software PCs 120 and 140. This is because it is possible to specify the most suitable software, for example, the latest software by visually observing the identifier of the software used by the service person. However, the time and effort of the service person can be omitted and it is possible to uniquely specify the attached software more accurately and reliably by providing the step S404.

In a case where only the main body software holds the identifier of the attached software, the processing is performed by omitting the identifier of the main body software from Input 401 of FIG. 5 and omitting the step S402 of FIG. 5.

In a case where only the attached software holds the identifier of the main body software, the processing is performed by omitting the identifier of the attached software from Input 401 of FIG. 5 and omitting the step S403 of FIG. 5.

However, as in the embodiment, the main body software holds the identifier of the attached software, the attached software holds the identifier of the main body software, and both of the step S402 and the step S403 of FIG. 5 are compared and specified. Accordingly, it is possible to specify the latest corresponding attached software and the corresponding attached software can be managed more strictly. The reason thereof will be described using Venn diagrams of FIGS. 6 and 7 described below.

FIG. 6 shows an image of the state in which the attached software corresponding to the identifier "02-01" is specified among the main body software shown as an example in FIG. 3 or 4.

In FIG. 6, the aggregation 501 is an aggregation of the attached software specified in the step S402 of FIG. 5, and the aggregation 502 is an aggregation of the attached software specified in the step S403 of FIG. 5. In this case, the aggregation 503 is an aggregation of the attached software specified by the both aggregation 501 and aggregation 502.

As shown in the aggregation 503, the attached software "03-01" is out of the aggregation 501, and the attached software "01-01" is out of the aggregation 502, and accordingly, they are not included in the aggregation 503.

In this case, the specific target in the processing of the step S404 is the latest attached software "02-02" in the aggregation 503.

FIG. 7 is a Venn diagram showing an image of the state of the attached software specified by the step S402 and the step S403 of FIG. 5, on the assumption that the attached software corresponding to the main body software is "01-02", but has not been provided yet, although the identifier "01-03" is provided as an updated version of the identifier "01-02" of the main body software shown as an example of FIG. 3 or 4.

In this case, as shown in FIG. 7, the aggregation 601 and the aggregation 602 have a sparse relationship and do not overlap each other. Thus, the corresponding attached software is not present. As such, by executing the both step S402 and the step S403, it is possible to specify the attached software more accurately.

Here, in a case of a return no specific result, the service person specifies the corresponding attached software by himself in the same manner as the related art, and it is desirable to execute maintenance work after inputting the device information on the automatic analyzer to be maintained.

A case is described, in which the storage location of the attached software specified by the flow chart of FIG. 5 is the recording devices 103a and 113a of the operation units 103 and 113, but a method of releasing the specified attached software is not particularly limited. For example, a file sharing method using a protocol such as FTP may be used, a method using a WEB server and an HTTP protocol may be used, or a method of directly outputting from the operation units 103 and 113 to an external recording medium or a recording medium of the attached software PCs 120 and 140 may also be used.

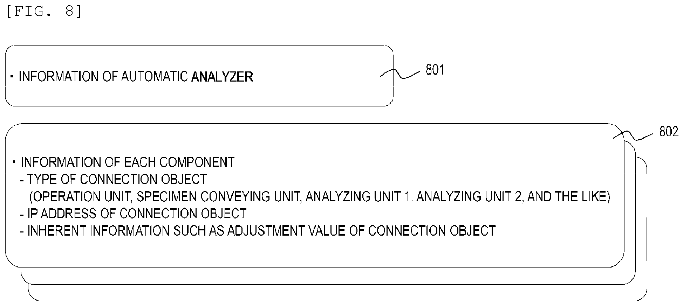

It is preferable that the operation units 103 and 113 record the attached software uniquely specified in the step S402 and the step S404 in the recording devices 103a and 113a, in a state where the device information of the automatic analyzers 100 and 110 to which the operation units 103 and 113 belong is reflected, and release the attached software in a state where the information is reflected. Hereinafter, the reflected information or reflection method will be described using FIGS. 8 to 10.

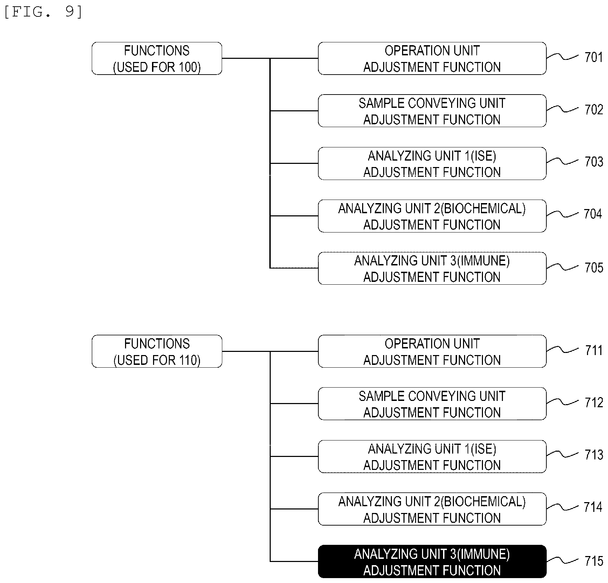

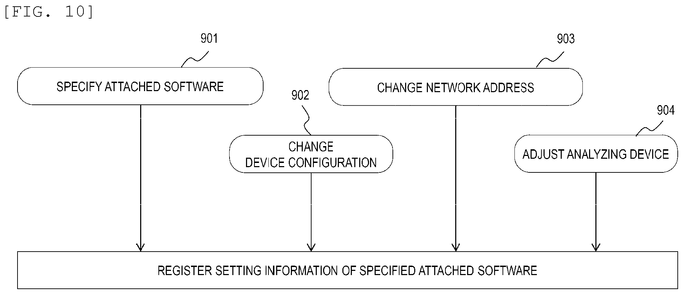

FIG. 8 is a diagram showing an example of the device information reflected to the attached software. FIG. 9 is a diagram showing an example of the functions of the attached software to validate or invalidate. FIG. 10 is a diagram showing an example of update timing of the setting information of the attached software.

The operation units 103 and 113 reflect the device information such as information 801 and 802 shown in FIG. 8 to the specified attached software. The information 801 is information that stores the information on the entire system such as names of the automatic analyzers 100 and 110. The information 802 is one example of the information corresponding to each component of the automatic analyzers 100 and 110, and holds the information for the number of the components. The information 801 and 802 are recorded in the recording devices 103a and 113a of the operation units 103 and 113.

The information 802 are configured by the information related to the type of connection objects such as the operation units 103 and 113, the sample conveying units 101 and 111, and the analyzing units 102a, 102b, 102c, 112a, and 112b, the information of IP addresses assigned to these connection objects, and the inherent information such as adjustment values of the connection objects.

It is desirable that the operation units 103 and 113 select functions suitable for the own machine from the information related to the type of connection objects and make the unnecessary function invalid. Hereinafter, the functions will be described using FIG. 9. FIG. 9 is one example of the functions of the attached software connected to the automatic analyzer 100 or the automatic analyzer 110 shown in FIG. 1.

The function 701 of FIG. 9 is a function corresponding to the operation unit 103 in the automatic analyzer 100 shown in FIG. 1, and transmits and/or receives information by communication with the operation unit 103. The function 702 of FIG. 9 is a function corresponding to the sample conveying unit 101 in the automatic analyzer 100 shown in FIG. 1, and adjusts the mechanism or transmits and/or receives information by communication with the sample conveying unit 101. The function 703 of FIG. 9 is a function corresponding to the analyzing unit 102a in the automatic analyzer 100 shown in FIG. 1, and adjusts the mechanism or transmits and/or receives information by communication with the analyzing unit 102a. The function 704 of FIG. 9 is a function corresponding to the analyzing unit 102b in the automatic analyzer 100 shown in FIG. 1, and adjusts the mechanism or transmits and/or receives information by communication with the analyzing unit 102b. The function 705 of FIG. 9 is a function corresponding to the analyzing unit 102c in the automatic analyzer 100 shown in FIG. 1, and adjusts the mechanism or transmits and/or receives information by communication with the analyzing unit 102c.

The function 711 of FIG. 9 is a function corresponding to the operation unit 113 in the automatic analyzer 110 shown in FIG. 1, and transmits and/or receives information by communication with the operation unit 113. The function 712 of FIG. 9 is a function corresponding to the sample conveying unit 111 in the automatic analyzer 110 shown in FIG. 1, and adjusts the mechanism or transmits and/or receives information by communication with the sample conveying unit 111. The function 713 of FIG. 9 is a function corresponding to the analyzing unit 112a in the automatic analyzer 110 shown in FIG. 1, and adjusts the mechanism or transmits and/or receives information by communication with the analyzing unit 112a. The function 714 of FIG. 9 is a function corresponding to the analyzing unit 112b in the automatic analyzer 110 shown in FIG. 1, and adjusts the mechanism or transmits and/or receives information by communication with the analyzing unit 112b.

On the other hand, the function 715 of FIG. 9 is a function corresponding to an immune analyzing unit, but the immune analyzing unit is not present in the automatic analyzer 110. For this reason, it is desirable that the operation unit 113 performs the processing of invalidating the function unnecessary for the own machine and reflects the information of each constituent machine before releasing the attached software. As such, the number of functions and the type of the attached software after being released vary depending on the constituent element and the type of the automatic analyzer to be connected.

The processing of reflecting the device information is only limited before releasing toward the attached software PCs 120 and 140.

As shown in FIG. 10, as the reflection timing, in addition to the above-described timing of specifying the attached software 901 of FIG. 5, device configuration change timing 902, network address change timing 903, analyzing system adjustment timing 904, and the like are exemplified.

As described above, the reflection timing is desirably the update timing of the information related to the setting information of the attached software, but is not particularly limited.

Here, the analyzing system adjustment timing 904 means a timing at which the operation units 103 and 113 receive position adjustment results of each mechanism within the analyzing units 102a, 102b, 102c, 112a, and 112b, and the adjustment results are reported to the operation units 103 and 113 via the network 104 and 114.

The attached software PCs 120 and 140 of the embodiment determine the information such as the number of functions, type, IP address of the connection object, and the like by referring to the device information of FIG. 8 registered at the timing of FIG. 10 when the attached software is started. Also, in a case where a mechanism of the analyzing unit to which the attached software is connected is adjusted, the mechanism is adjusted using the adjustment result registered by the main body software as an initial value.

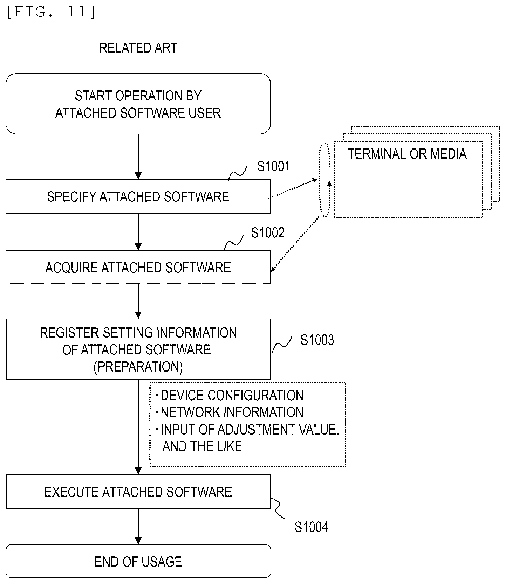

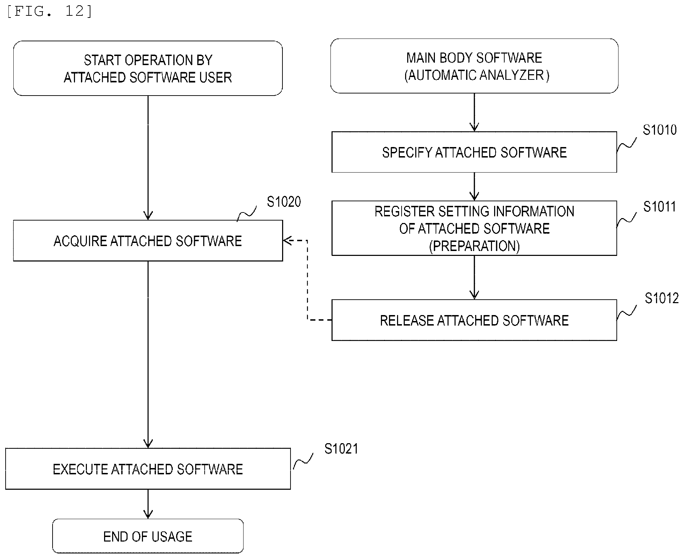

Hereinafter, FIGS. 11 and 12 will describe how a workflow of the service person differs between the automatic analyzer in the related art and the automatic analyzer of the embodiment. FIG. 11 is a diagram showing a workflow for maintenance of the automatic analyzer in the related art, and FIG. 12 is a diagram showing a workflow for maintenance of the automatic analyzer of the embodiment.

In order to utilize the attached software in the automatic analyzer in the related art, as shown in FIG. 11, after starting the work, a user of the attached software initially specifies the attached software corresponding to the automatic analyzer from a terminal or media while firstly looking at the correspondence table as shown in FIG. 4 (step S1001), the attached software is obtained to the attached software PC (step S1002), and after the setting information corresponding to the automatic analyzer to be used is registered (step S1003), the attached software is executed (step S1004) . As described above, time and effort are required before starting the maintenance work.

On the other hand, in the automatic analyzer of the embodiment, it is not necessary to execute the step S1001, the step S1002, and the step S1004 which have been performed in the related art by a user of the attached software. The operation units 103 and 113 of the automatic analyzer specify the attached software (step S1010), register the setting information of the attached software (step S1011), and release the attached software (step S1012).

For this reason, the processing to be executed at the time of utilizing the attached software are only two steps, which are obtaining the specified attached software to the attached software PCs 120 and 140 (step S1020) and executing the specified attached software (step S1021). Accordingly, the work of the user of the automatic analyzer of the invention is considerably simplified compared to the related art, and a risk of using the incorrect attached software can be also reduced compared to the related art.

For example, in a case where the automatic analyzer 100 and the automatic analyzer 110 whose device configuration shown in FIG. 1 is different from each other are different identifiers, the user of the automatic analyzer in the related art needs to specify the attached software corresponding to each of the automatic analyzer 100 and the automatic analyzer 110 and register the setting information of the attached software.

However, in a case of the automatic analyzer of the embodiment, the attached software corresponding to the automatic analyzer 100 is automatically obtained from the operation unit 103, and the attached software corresponding to the automatic analyzer 110 is automatically obtained from the operation unit 113. Thus, the attached software can be used without registering the setting information.

Next, the effect of the embodiment will be described.

According to the above-described automatic analyzers 100 and 110 of the embodiment, a user of the attached software can use the appropriate and correct attached software without comparing the version compatibility of the attached software or the main body software of the automatic analyzer. For this reason, the service person can quickly provide high quality maintenance work or adjustment work service to customers. Also, in the related art, there was no guarantee that the service person can always select the suitable attached software, but according to the invention, the risk of selecting such inappropriate attached software can be eliminated as much as possible.

Further, in a case where the main body software or the attached software only holds the corresponding identification information, the processing necessary for specifying can be simplified.

On the contrary, in a case where both the main body software and the attached software hold the corresponding identification information, the appropriate attached software can be specified even in a case where a plurality of pieces of attached software is specified, and the quality of the maintenance work can be further improved.

Moreover, in a case where a plurality of pieces of attached software is specified as a result of comparing the specific identification information and the corresponding identification information, the operation units 103 and 113 can uniquely select the most appropriate attached software more accurately and reliably, by selecting the most appropriate attached software among the plurality of pieces of attached software, especially selecting the latest attached software.

The operation units 103 and 113 reflect the device information of the automatic analyzers 100 and 110 to which the operation units 103 and 113 belong to the specified attached software. Accordingly, the service person can start the maintenance work without preliminary preparation based on the inherent information of the object automatic analyzer at the time of using the attached software, and burdens such as the setting of the attached software, and the like can be further reduced.

The device information is at least one of the information on each constituent machine, IP addresses thereof, and inherent adjustment value of each machine, so that the work of investing various inherent information of the device or the work of inputting the information accurately, which are great burdens for the service person, can be omitted, and the burden on the service person can be further reduced.

When the power supply of the automatic analyzers 100 and 110 are turned ON, regardless of the execution of maintenance, the operation units 103 and 113 execute the processing of specifying the attached software to prepare the appropriate version of the attached software in advance in the automatic analyzers 100 and 110 at the timing when the maintenance can be executed. Accordingly, the appropriate attached software can be provided quickly after the attached software PCs 120 and 140 are connected.

The automatic analyzers 100 and 110 further include the recording devices 103a and 113a which record the attached software, and the operation units 103 and 113 record the attached software reflecting the device information of the automatic analyzers 100 and 110 in the recording devices 103a and 113a. Accordingly, even if the information managing PC 130 or network 131 is out of order, maintenance or adjustments can be executed by using the appropriate and latest attached software recorded in the recording devices 103a and 113a by the operation units 103 and 113, and accurate maintenance service can be provided even if unexpected situation occurs.

The operation units 103 and 113 release the specified attached software toward the attached software PCs 120 and 140 operated by the service person, and accordingly the appropriate attached software can be easily obtained from the automatic analyzers 100 and 110 at the time of accessing from the attached software PCs 120 and 140, and further labor can be saved reliably and accurate maintenance work or adjustment work can be provided.

Even when change of the network address, change of the device configuration, and adjustment of the analysis device are completed, the operation units 103 and 113 reflect the above information to the device information of the automatic analyzers 100 and 110 and record the information in the recording devices 103a and 113a. Accordingly, the attached software recorded in the automatic analyzers 100 and 110 can be made more appropriate rather than the current device, and further accurate maintenance work and adjustment work can be provided.

### <Others>

The invention is not limited to the above-described embodiment, and various modifications and applications can be made. The above-described embodiment is a detailed description for explaining the invention more easily and is not necessarily limited to the aspect of including all of the explained configurations.

For example, the processing of specifying the attached software by the operation units 103 and 113 of the invention or various subsidiary processing can be executed by renewing the main body software of the object automatic analyzer, even with respect to the automatic analyzer that has been already used by customers. At that time, the main body software will be version-upgraded.

### Reference Signs List

- 9: reaction disk
- 10: reaction container
- 11: reagent disk
- 12: reagent bottle
- 13: specimen dispensing probe
- 14: stirring device
- 15: cleaning device
- 16: light source
- 17: multi-wavelength photometer
- 18: reagent dispensing probe
- 19: rack conveying belt
- 22: interface
- 23: computer
- 24: A/D converter
- 100, 110: automatic analyzer
- 101, 111: sample conveying unit
- 101a, 111a: specimen rack
- 101a1, 111a1: specimen container
- 102a, 102b, 102c, 112a, 112b: analyzing unit
- 103, 113: operation unit (control unit)
- 103a, 113a: recording device
- 104, 114: network
- 120, 140: attached software PC (computer operated by service person)
- 130: information managing PC
- 130a: recording device
- 131: network
- 201: example
- 501, 502, 503, 601, 602: aggregation
- 701, 702, 703, 704, 705, 711, 712, 713, 714, 715: function of attached software
- 801, 802: setting information of attached software
- 901, 902, 903, 904: setting update timing of attached software

## Claims

1. An automatic analyzer comprising:
an analyzing unit that executes specimen analysis; and
a control unit that controls operation of machines in the device including the analyzing unit, wherein
the automatic analyzer is operable by main body software that controls operation of the automatic analyzer and attached software that is independent of the main body software and is used during maintenance of the machines that configure the automatic analyzer,
the attached software and the main body software respectively hold specific identification information that specifies an own version,
at least one of the attached software and the main body software holds corresponding identification information of a counterpart side software corresponding to current time, and
the control unit specifies the attached software suitable for the main body software from a plurality of pieces of attached software by comparing the specific identification information and the corresponding identification information.

2. The automatic analyzer according to claim 1, wherein only the main body software has the corresponding identification information.

3. The automatic analyzer according to claim 1, wherein only the attached software has the corresponding identification information.

4. The automatic analyzer according to claim 1, wherein both the main body software and the attached software have the corresponding identification information.

5. The automatic analyzer according to claim 1, wherein when a plurality of pieces of attached software are specified as a result of comparing the specific identification information and the corresponding identification information, the control unit selects a most suitable attached software for the main body software from the plurality of pieces of attached software.

6. The automatic analyzer according to claim 5, wherein the control unit selects a latest attached software as the most suitable attached software for the main body software.

7. The automatic analyzer according to claim 1, wherein the control unit reflects device information of the automatic analyzer to which the control unit belongs to the specified attached software.

8. The automatic analyzer according to claim 7, wherein the device information is at least one of information on the machines constituting the device, IP addresses of the machines, and inherent adjustment values of the machines.

9. The automatic analyzer according to claim 1, wherein the control unit executes a specification processing for the attached software when a power supply of the automatic analyzer is turned ON.

10. The automatic analyzer according to claim 9, wherein the control unit reflects device information of the automatic analyzer to which the control unit belongs to the specified attached software.

11. The automatic analyzer according to claim 10, further comprising:
a recording device that records the attached software, wherein
the control unit records the attached software reflecting the device information of the automatic analyzer in the recording device.

12. The automatic analyzer according to claim 11, wherein the control unit also reflects information on the device information of the automatic analyzer and records the reflected information in the recording device when a network address changes, a device configuration changes, and analyzer adjustment are completed.

13. The automatic analyzer according to claim 1, wherein the control unit releases the specified attached software to a computer operated by a service person.
